# FASCICULE DE BREVET EUROPEEN

(11) **EP 0 311 540 B1**
(45) Date de publication et mention de la délivrance du brevet: **12.01.1994**
(21) Numéro de dépôt: 88420340.7
(22) Date de dépôt: 07.10.1988
(51) Int. Cl.: A61K 31/57, A61K 9/18, A61K 9/20

(54) **Nouvelles formes galéniques de corticoides pour administration par voie per- et sublinguale et leur procédé de préparation**
Galenische Kortikoidformen für die per- und sublinguale Verabreichung und ihr Herstellungsverfahren
Galenical forms of corticoids for per- and sublingual administration, and their preparation process

(30) Priorité: 08.10.1987 FR 8714476
(43) Date de publication de la demande: 12.04.1989
(73) Titulaire: MEDIBREVEX SA, 74940 Annecy Le Vieux (FR)
(72) Inventeur: Clairet, épouse Ayache, Josiane, F-38000 Grenoble (FR); Ayache, Jean-Jacques, F-38000 Grenoble (FR); Bruttmann, Georges, F-38000 Grenoble (FR); Pedrali, Patrick, F-74000 Annecy (FR); Robert, Serge, B-1440 Braine le Château (BE)
(74) Mandataire: Maureau, Philippe

(56) Documents cités:
- EP-A- 0 205 282
- CH-A- 156 609
- FR-A- 2 285 896
- US-A- 4 259 314
- M. Traisnel et al., "GALENICA", vol. 16, "Médicaments Homéopathiques", 2ème édition, 1986, Technique et Documentation, Paris (FR); J. ABECASSIS, pp. 77-99#

## Description

La présente invention concerne de nouvelles formes galéniques de corticoïdes pour administration par voie per- et sublinguale ainsi que leur procédé de préparation.

Après les travaux princeps de CARRYER en 1950, de nombreux travaux ont confirmé les effets bénéfiques des glucocorticoïdes en thérapeutique humaine. Les glucocorticoïdes utilisés sont des dérivés synthétiques.

Les corticoïdes sont préconisés surtout pour leurs effets anti-inflammatoires, ce qui permet leur utilisation dans un vaste domaine de la pathologie humaine.

Les glucocorticoïdes de synthèse ont des propriétés différentes du cortisol surrénalien humain : une plus grande diffusion dans les tissus, un métabolisme hépatique plus lent qui explique l'allongement de leur demi-vie. Ils sont classés selon la durée de leurs effets biologiques en glucocorticoïdes d'action courte (inférieure à 18 heures), d'action intermédiaire (18 heures à 36 heures), d'action longue (36 heures à 54 heures).

Le choix du corticoïde se fait donc en fonction de sa forme galénique, de son activité anti-inflammatoire, de sa faible activité minéralo-corticoïde, et d'un effet freinateur hypophysaire court.

L'administration par voie classique des glucocorticoïdes conduit souvent à un certain nombre d'incidents : candidoses bucco-pharyngées, atrophie dermo-hypodermique au point d'injection, troubles gastro-intestinaux, accidents métaboliques, ostéoporose, risques infectieux.

Conformément au document EP-A-0 205 282, on a décrit une forme dite "buccale" d'un cortico-stéroïde. A l'examen attentif de la description de ce document, cette forme buccale est en fait une forme orale, à effet retardé du cortico-stéroïde, et donc avec passage de ce dernier par le tractus gastro-intestinal.

En effet, selon ce document, le principe actif fait partie d'un support solide, en l'occurrence des granules, formulées pour adhérer à la muqueuse de la bouche.

Conformément au document US-C-4,259,314, on a décrit également une forme orale, à effet retardé, c'est-à-dire avec une libération progressive du principe actif. Cette forme a pour support un excipient obtenu à partir de hydroxypropyl-méthylcellulose et d'hydroxypropyl cellulose.

Conformément au document FR-A-2 285 896, on a aussi décrit une forme orale, à effet retardé, d'un principe actif.

Tout support adhérant à la muqueuse de la bouche, pour une libération progressive ou contrôlée du principe actif, provoque en permanence une hypersalivation, laquelle transfère quasi-immédiatement le principe actif par la voie orale, au fur et à mesure de sa libération, le patient avalant en permanence sa salive. Or le passage d'un corticoïde par le tractus gastro-intestinal, et donc par la voie hépatique, métabolise en partie ce principe actif, en altérant pour une grande part son efficacité thérapeutique.

La présente invention a pour objet une forme galénique de corticoïdes, évitant en grande partie le tractus gastro-intestinal.

Conformément à la présente invention, les inventeurs ont pu déterminer, de façon surprenante, qu'il était possible peur des corticoïdes d'éviter la voie hépatique, en choisissant une forme galénique adaptée à une administration sublinguale et perlinguale desdits corticoïdes.

Un médicament à base de corticoïdes selon l'invention est caractérisé en ce qu'il consiste en une composition solide divisée pour une administration subliguale et perlinguale dudit corticoïde, permettant une absorption rapide de ce dernier sans déglutition, comprenant d'une part un support solide dans le groupe comprenant le saccharose, le lactose et leur mélange, et d'autre part, ledit corticoïde intégré audit support.

Cette voie per- et sublinguale est utilisée à cause de sa configuration anatomique, qui en fait une entité par rapport aux autres éléments de la cavité buccale.

Schématiquement, on peut décrire la région sublinguale de la manière suivante (chacun des éléments constitutifs ayant des propriétés utiles dans l'administration des médicaments introduits par cette voie).

Globalement, la région sublinguale comprend :
- la face inférieure de la langue qui sert de plafond, très riche en vaisseaux sanguins : veines, artères et vaisseaux lymphatiques ;
- le plancher de la bouche qui constitue la partie inférieure de la région sublinguale. C'est également une région anatomique qui comporte beaucoup de vaisseaux sanguins et surtout un très riche réseau veineux, des artères et des vaisseaux lymphatiques ;
- les bords de la région sublinguale sont formés par les parties montantes du maxillaire inférieur, les gencives et les dents.

Dans le plancher de la bouche, on trouve des glandes salivaires, les glandes sublinguales, qui secrètent de la salive dès qu'il existe un contact avec la région sublinguale. Par sa composition, la salive participe activement au délitement du produit pharmacologiquement actif présenté sous cette forme galénique sublinguale particulière selon l'invention.

Par ailleurs, la structure histologique montre l'existence de cellules immuno-compétentes en grand nombre qui vont, dans certains cas, favoriser l'activité médicamenteuse.

Par rapport aux autres constituants de la bouche : intérieur des joues, partie supérieure de la langue, la région sublinguale apparaît donc comme une entité anatomique particulière qui en fait une voie d'administration médicamenteuse privilégiée comparable à l'administration des médicaments par voie injectable.

Tous les autres médicaments dont le délitement se fait dans la bouche sont forcément avalés.

Dans la forme galénique sublinguale selon l'invention, l'activité du produit est fondée sur sa possibilité d'absorption rapide sans déglutition. L'avantage que présente cette forme galénique par rapport aux autres formes galéniques utilisées à l'intérieur de la cavité buccale est que le produit passe directement dans le sang, ce qui évite le métabolisme hépatique; le produit agit donc plus rapidement avec une efficacité maximale.

Les incidents décrits ci-avant sont évités et il est ainsi possible d'administrer des doses très faibles avec un très bon résultat clinique; l'absorption se fait très rapidement et équivaut à une injection intraveineuse, voire intramusculaire.

Le passage perlingual et sublingual de corticoïdes apparaît surprenant, pour plusieurs raisons :
a) conformément à une littérature scientifique constante, il est admis que tout principe actif n'est pas en soi transférable par la muqueuse sublinguale
b) en particulier, on connaît mal les facteurs qui sont favorables à ce passage, tels que pH, caractère lipophile, caractère hydrophile, pk_{A}, taille de la molécule, etc...
c) c'est le mérite de l'invention d'avoir mis en évidence la possibilité de passage sublingual de différents corticoïdes
d) compte tenu de l'encombrement stérique des molécules des principaux corticoïdes, on pouvait plutôt s'attendre à un passage limité des corticoïdes par la paroi sublinguale.

Ce passage sublingual apporte un avantage déterminant pour les corticoïdes. Si on connaît l'efficacité clinique de ces substances, on connaît aussi leur toxicité. Leur passage direct dans le sang par la voie sublinguale permet de limiter les quantités administrées, et donc la toxicité.

Conformément à l'ouvrage suivant : M. Traisnel et al. "GALENICA", vol 16, "Médicaments homéopathiques", 2ème édition, 1986, pages 77-99, Technique et Documentation, Paris, on a décrit différentes formulations solides destinées à la cavité sublinguale pour des principes homéopathiques.

Jusqu'à présent, allopathie et homéopathie s'opposent, et il n'est guère souvent venu à l'esprit d'un thérapeute allopathe d'aller chercher des solutions dans les manuels d'homéopathie... En particulier, l'homéopathie opère avec des quantités du principe actif, ou ce qui en tient lieu, qui sont extrêmement diluées (cf dilutions de Korsakoff ou Hahnemann), au point de ne plus savoir si ce principe actif existe encore sur le support retenu, ou de ne plus être en mesure de doser et donc contrôler cette quantité de principe actif. Selon l'invention, la quantité de principe actif existe et reste contrôlée et contrôlable.

La nouvelle forme galénique selon l'invention ne s'adresse qu'aux traitements ambulatoires ; elle est destinée aussi bien aux enfants qu'aux adultes.

Le procédé d'obtention des nouvelles formes galéniques selon l'invention va maintenant être décrit en détail ; le corticoïde retenu est la méthylprednisolone.

On procède d'abord à la mise en solution aqueuse des produits lyophylisés de façon à obtenir une dilution à 5 mg de méthylprednisolone ou toutes autres concentrations si cela se révèle nécessaire. On peut également se servir de la forme liquide pour imprégner directement les globules.

La solution "mère" peut être obtenue avec tout autre solvant convenable de façon à obtenir une solution mère de concentration élevée. On peut par exemple utiliser un autre solvant polaire tel que le sérum physiologique ou l'alcool éthylique de titre faible.

On peut, à partir de cette solution "mère", préparer d'autres concentrations à la demande, en utilisant le même solvant que celui qui a servi pour préparer celle-ci.

Les solutions aux différentes concentrations voulues sont incorporées à un excipient neutre, en l'occurence, soit du saccharose, soit du lactose, soit encore un mélange des deux. Cette dernière possibilité est utilisée sous une forme pharmaceutique particulière, le globule ou le granule.

Ces formes pharmaceutiques sont de petites sphères de saccharose (85 %) et de lactose (15 %) telles qu'il en faut 20 pour faire un gramme pour les granules et environ 200 pour les globules.

Un gramme de granules ou de globules se différencie donc notamment par la surface sphérique totale, plus importante pour les globules puisque plus nombreux.

Cette grande surface sert de support d'imprégnation pour les substances actives.

Les globules sont fabriqués par dragéification de cristaux de sucre que l'on enrobe progressivement de couches concentriques de saccharose et de lactose.

Le point de départ est constitué par un sucre cristallisé calibré, tel que 10 000 cristaux de sucre pèsent un gramme. Ces cristaux de sucre sont placés dans des turbines à dragéifier en acier inoxydable et soumis à un enrobage progressif.

Chaque cycle d'enrobage comporte quatre opérations successives :
- pulvérisation sur la masse en turbine d'un sirop de sucre ;
- mélange de la masse et du sirop pulvérisé ;
- poudrage de la masse par un mélange de surcre glace et de lactose ;
- séchage de la masse par insufflation d'air chaud.

Ce cycle de fabrication est répété une multitude de fois, jusqu'à l'obtention de globules dont le poids est de 50 fois supérieur au poids de départ des cristaux de sucre.

Les quantités de sirop pulvérisées à chaque cycle, la concentration et la température du sirop, la pression de pulvérisation, le temps de mélange, la quantité de poudrage et le temps de séchage sont parfaitement codifiés et varient d'un stade à l'autre de la fabrication.

Après chaque journée de travail en turbine, il est nécessaire d'étaler les grains sur des plateaux en inox sous faible épaisseur et de les laisser séjourner quelques heures dans une étuve ventilée à 30-35° pour obtenir des grains à taux d'humidité convenable avant de poursuivre l'enrobage. Un calibrage par tamisage est également effectué quotidiennement.

Il est évident que, sans sortir du cadre de l'invention, ces globules peuvent être remplacés par des poudres ou des comprimés, également adaptés à l'application perlinguale ou sublinguale.

Ces globules neutres sont rendus actifs par la technique d'imprégnation. Cette dernière se réalise tout simplement en agitant les globules en rotation dans une turbine avec la quantité de dilution adéquate du produit à imprégner ; celui-ci est incorporé aux globules à l'aide d'un injecteur du type dénommé "spray-doseur".

Pour assurer une homogénéisation parfaite, on réalise une imprégnation fractionnée consistant à injecter la totalité du produit actif en 3 à 5 fois.

Entre chaque imprégnation, le séchage est réalisé par passage d'air forcé et désséché à une température inférieure à 35°C.

Selon le type de substance à imprégner, on peut avoir intérêt à travailler sous atmosphère d'azote afin de garantir que soit totalement maintenue l'intégrité physico-chimique du principe actif.

On peut envisager également, au stade final de l'imprégnation, d'enrober le globule d'une dernière couche sucrée afin d'isoler totalement le principe actif.

## Revendications

1. Médicament à base de corticoïdes, **caractérisé en ce qu'**il consiste en une composition solide divisée pour une administration sublinguale et perlinguale dudit corticoïde, permettant une absorption rapide de ce dernier sans déglutition, comprenant, d'une part, un support solide choisi dans le groupe comprenant le saccharose, le lactose et leur mélange, et d'autre part, ledit corticoïde intégré audit support.

2. Médicament selon la revendication 1, caractérisé en ce que le support solide est un mélange de saccharose et lactose.

3. Médicament selon la revendication 2, caractérisé en ce que le support solide comprend 85 % de saccharose et 15 % de lactose.

4. Procédé de préparation d'un médicament selon l'une quelconque des revendications 1 à 3, caractérisé en ce qu'il comporte les étapes suivantes :
- mise en solution du corticoïde dans un solvant pour obtenir une solution mère ;
- préparation de dilutions à différentes concentrations, à partir de la solution mère ;
- fractionnement de chacune de ces dilutions en sous-dilutions ;
- imprégnation par multi-imprégnation ou imprégnation fractionnée de support solide pharmaceutiquement acceptable avec chacune des sous-dilutions, chacune desdites étapes d'imprégnation étant suivie d'un séchage à air forcé et desséché à une température inférieure ou égale à 35 °C ;
- éventuellement imprégnation finale protectrice du type dragéification.

## Claims

1. Corticoid-based medicinal product, characterised in that it consists of a divided solid composition for sublingual and perlingual administration of the said corticoid, permitting a rapid absorption of the latter without swallowing, comprising, on the one hand a solid vehicle chosen from the group comprising sucrose, lactose and a mixture thereof, and on the other hand the said corticoid integrated in the said vehicle.

2. Medicinal product according to Claim 1, characterised in that the solid vehicle is a mixture of sucrose and lactose.

3. Medicinal product according to Claim 2, characterised in that the solid vehicle comprises 85% of sucrose and 15% of lactose.

4. Process for preparing a medicinal product according to any one of Claims 1 to 3, characterised in that it entails the following steps:
- dissolution of the corticoid in a solvent to obtain a stock solution;
- preparation of dilutions at different concentrations, from the stock solution;
- fractionation of each of these dilutions into subdilutions;
- impregnation by multi-impregnation or fractional impregnation of a pharmaceutically acceptable solid vehicle with each of the subdilutions, each of the said impregnation steps being followed by a drying with forced air dried at a temperature not exceeding 35°C;
- where appropriate, protective final impregnation of the sugar-coating type.

## Patentansprüche

1. Arzneimittel auf Basis von Kortikoiden, dadurch gekennzeichnet, daß es aus einer festen Zusammensetzung besteht, die für eine sublinguale und eine perlinquale Verabreichung des genannten Kortikoides aufgegliedert ist, wodurch eine rasche Resorption des letzteren ohne einen Schluckakt möglich ist, wobei die Zusammensetzung einesteils einen festen Träger, ausgewählt aus der Gruppe bestehend aus Saccharose, Lactose und deren Mischung, und andernteils das genannte Kortikoid im Träger integriert aufweist.

2. Arzneimittel nach Anspruch 1, dadurch gekennzeichnet, daß der feste Träger eine Mischung von Saccharose und Lactose ist.

3. Arzneimittel nach Anspruch 2, dadurch gekennzeichnet, daß der feste Träger 85 % Saccharose und 15 % Lactose enthält.

4. Verfahren zum Herstellen eines Arzneimittels nach einem der Ansprüche 1 bis 3, dadurch gekennzeichnet, daß es die folgenden Schritte aufweist:
- Lösen des Kortikoides in einem Lösungsmittel zum Erhalten einer Stammlösung;
- Herstellen von Verdünnungen mit unterschiedlichen Konzentrationen aus der Stammlösung;
- Fraktionieren jeder der Verdünnungen in Unterverdünnungen;
- Imprägnieren eines pharmazeutisch verträglichen festen Trägers durch Multi-Imprägnieren oder durch fraktionierte Imprägnierung mit jeder der Unterverdünnungen, wobei jede der Imprägnierungsstufen von einer Trocknung mit bewegter und getrockneter Luft bei Temperaturen unterhalb oder bei 35°C gefolgt ist;
- ggf. abschließende Schutz-Imprägnierung in Art einer Dragéebildung.
